(19) Europäisches Patentamt

European Patent Office

Office européen des brevets

(11) EP 0 730 873 B1

(12) **EUROPEAN PATENT SPECIFICATION**

(45) Date of publication and mention
of the grant of the patent:
**16.08.2001 Bulletin 2001/33**

(51) Int Cl.$^7$: **A61K 51/12**

(21) Application number: **96400477.4**

(22) Date of filing: **06.03.1996**

(54) **A radioactive patch/film and process for preparation thereof**

Ein radioaktives Pflaster/Film und Verfahren zu seiner Herstellung

Patch/film radioactif et son procédé de préparation

(84) Designated Contracting States:
**AT BE CH DE DK ES FI FR GB GR IE IT LI LU MC NL PT SE**

(30) Priority: **07.03.1995 KR 9504642**
**29.02.1996 KR 9605322**

(43) Date of publication of application:
**11.09.1996 Bulletin 1996/37**

(73) Proprietor: **KOREA ATOMIC ENERGY RESEARCH INSTITUTE**
**Daejeon-si (KR)**

(72) Inventors:
 • **Park, Kyoung-Bae**
 **Yongsan-ku, Seoul (KR)**
 • **Kim, Jae-Rock**
 **Sungdong-Ku, Seoul (KR)**
 • **Lee, Jong-Du**
 **Yangchun-Ku, Seoul (KR)**

(74) Representative: **Boulinguiez, Didier**
**Cabinet Plasseraud**
**84, rue d'Amsterdam**
**75440 Paris Cedex 09 (FR)**

(56) References cited:
**BE-A- 514 504          DE-A- 4 132 925**
**FR-A- 1 458 937        GB-A- 253 207**
**GB-A- 704 628          US-A- 4 789 501**
**US-A- 4 946 435**

• **CHEMICAL ABSTRACTS, vol. 123, no. 22, 27 November 1995 Columbus, Ohio, US; abstract no. 296586, KAWASHITA, M. ET AL: "Preparation of phosphorus-containing silica glass for radiotherapy by co-implantation of phosphorus and nitrogen ions" XP002007324 & J. MATER. SCI.: MATER. MED. (1995), 6(10), 606-11 CODEN: JSMMEL;ISSN: 0957-4530, 1995,**
• **CHEMICAL ABSTRACTS, vol. 76, no. 19, 8 May 1972 Columbus, Ohio, US; abstract no. 109734, KIR'YAKOV, M. A. ET AL: "Use of palladium-103 films dissolving in the organism for the treatment of experimental tumors" XP002007325 & MED. RADIOL. (1972), 17(2), 50-2 CODEN: MERAA9, 1972,**
• **DATABASE EMBASE ELSEVIER SCIENCE PUBLISHERS, AMSTERDAM, NL AN=75149007, XP002007327 & MED. RADIOL. , vol. 19, no. 11, 1974, (MOSK.), pages 30-35, DEDENKOV A. I. ET AL.: "USE OF DISOLVING RADIOACTIVE PREPARATIONS IN EXPERIMENTAL ONCOLOGY."**
• **CHEMICAL ABSTRACTS, vol. 76, no. 19, 8 May 1972 Columbus, Ohio, US; abstract no. 109734, KIR'YAKOV, M. A. ET AL: "Use of palladium-103 films dissolving in the organism for the treatment of experimental tumors" XP002007326 & MED. RADIOL. (1972), 17(2), 50-2 CODEN: MERAA9, 1972,**
• **J. Mater. Sci.:Mater. Med., Vol. 66, No. 10, 27 Nov 1995, pages 606 to 611, Kawashita M et al**

## EP 0 730 873 B1

**Description**

<u>Technical Field</u>

The present invention relates to a radioactive patch/film, a novel type for irradiating in the field of an external radiation therapy, and process for preparation thereof.

In particular, the present invention relates to a patch/film, which can be attached to patient's skin or mucous membrane to treat the lesions by direct irradiation.

And the present invention relates to the process for preparation of a radioactive patch/film, wherein 1) to prepare the radioactive patch; particles of stable nuclide mixed with adhesive agent are coated on the carrier, laminated for sealing, and then irradiated with neutrons in the nuclear reactor and 2) to prepare the radioactive film; the solution of stable nuclide mixed with the solution of adhesive agent are made into the thin membrane, dried, laminated for sealing and then irradiated with neutrons in the nuclear reactor.

<u>Background of Art</u>

There are two kinds of radiation therapy; one is the internal radiation therapy that radioactive material is administered into the lesion to emit radiation in the human body, and the other is the external radiation therapy that radiation is emitted from the outside of the body through special equipments.

In the internal radiation therapy, radioactive materials are administered orally, intravenously or by injecting directly into the body. But the internal radiation therapy has not yet been utilized widely on the reason that a radioactive material administered to the lesion may be leaked out and spread to the whole body through blood flow, and accumulated to other organs or tissues, and it results in a fatal damage to other organs, especially bone marrow very sensitive to radiation. Therefore, most malignant tumors have been treated by the external radiation therapy.

By the way, the external radiation also has many problems. For the external radiation therapy, radionuclides with high penatrative radiation should be used, since the rays are emitted out of the body, and it results in the irradiation of adjacent normal tissue as well as the lesion. And it is not convenient to use the external radiation on account of expensive equipments, hospitalization and the like.

US 4,946,435 describes a flexible sealed radioactive film for medical treatments comprising a radioactive material coated on or impregnated in a carrier film and sealed in an envelope. The carrier film is made of a material which can selectively trap the radioactive material while impureties are not retained. In a preferred embodiment, a membrane filter having uniform pore size is used as a carrier film, and a radio-labeled polyaminoacid is coated thereon.

The inventors have conducted intensive research in order to find the way of irradiating the lesion conveniently and invented the new therapeutic type for irradiation to patients. That is, radioactive material is prepared in a form of patch/film which makes irradiation easy and irradiates lesion site specifically.

<u>Summary of the invention</u>

The object of the present invention is to provide the radioactive patch/film for the external radiation therapy.

The radioactive patch of the present invention is prepared by the process, wherein the particles of stable nuclide mixed with adhesive agent are coated on the carrier, laminated for sealing to form the patch, and then irradiated with neutrons in the nuclear reactor to convert the patch into the radioactive patch.

The radioactive film of the present invention is prepared by the process, wherein the stable nuclide mixed with the solution of adhesive agent are made into the thin membrane, dried, laminated for sealing to form the film, and then irradiated with neutrons in the nuclear reactor to convert the film into the radioactive film.

And the object of the present invention is to provide the use of the radioactive patch/film as pharmaceutical agent for the external radiation therapy. The radioactive patch/film is attached to skin or mucous membrane and it irradiates lesion site directly.

And the object of the present invention is to provide the radioactive patch/film which irradiates only the lesion site without damage of the adjacent normal tissue, since the shape and the size of the patch/film are controlled according to those of the lesion.

And the object of the present invention is to provide the radioactive patch/film in which the irradiation dose can be controlled by amount of particles and their radioactivity.

The present invention will be described in detail in the following.

<u>Disclosure of the Invention</u>

The representative diseases which can be treated with the radioactive patch/film of the present invention are skin diseases. In the following, the radioactive patch/film will be described in detail in the view of treatment of dermal disease, especially skin cancer. But it is evident this patch/film can be used for other diseases which can be treated by the radiation therapy.

Skin cancer is a popular disease to prime age and old age, and it is developed by exposing to sunlight, pollution material, carcinogen, radiation and the like and also by chronic skin ulcer, burn and the like. The incidence of cancer disease is dependent on races, individuals and nations, but high in white people. For example, it is reported that in Canada skin cancer is developed in 39 persons out of 100,000 persons. For the treatment of skin cancer, one of the most common malignant tumor to human beings, lesion burning, irradiation or surgical therapy are often used.

But the surgical therapy of skin cancer has some troubles. The surgical therapy is impossible when the cancer is developed in the site impossible to be operated. Even if the surgical therapy is possible, the radiation therapy is preferred than the surgical therapy for skin cancer, since the skin cancer is often developed in multiple sites, and skin-grafting accompanied after surgical operation are not easy.

The effect of the radiation therapy is affected remarkably by total irradiation dose, divisional radiation, radiation range, property of radiation and the like.

Hitherto, X-rays, megavoltage electron rays or γ-ray has been used in the treatment of malignant skin cancer. Generally 50-100 Gy (5,000-10,000 rad) is optimal as total irradiation dose and this total dose should be divided and irradiated by each 2-3,5 Gy (200-350 rad) for about 4-6 weeks. The reason to irradiate the total irradiation dose in divisional pattern is to minimize the damage to the adjacent normal tissue or organ by X-rays, electron rays, γ-rays of high energy. The external radiation therapy has been widely used, since it leads to relatively good results, but there remains some demerits. Long-reaching radiation tends to damage adjacent organs such as bones, cartilage and the like, and the treatment takes long on account of divisional irradiation and has some difficulties because of expensive equipments and hospitalization.

Therefore the inventors of the present invention have completed this invention to overcome the demerits aforementioned. That is, to prepare the radioactive patch, particles of stable nuclide emitting high-energy(1.4-2.2MeV) β-ray or γ-ray are mixed with adhesive agent, coated on the carrier, laminated with polyethylene film for sealing, and then irradiated with neutrons in the nuclear reactor. And to prepare the radioactive film, the stable nuclide emitting β-ray or γ-ray is mixed with the solution of adhesive agent, made into thin membrane, dried, laminated with polyethylene film for sealing and then irradiated with neutrons in the nuclear reactor.

The radioactive patch/film of this invention has many merits to treat dermal diseases as described below. The radioactive patch/film uses β-rays, differently from previously established method using X-rays, electron rays and γ-rays, and hence adjacent organs, especially bone or bone marrow, are safe in spite of irradiation. And as the total irradiation dose, 50-100 Gy (5,000-10,000 rad) can be irradiated at one time. Therefore the period of irradiation reduces to 1-2 hrs, compared with about 4-6 weeks of the previous method and the period of attending hospital also reduces much. The used radionuclide decays soon due to short half-life of β-emitter such as 1-3 days.

Skin cancer developed in multiple sites can be treated simultaneously by using the radioactive patch/film of the present invention. Since the shape and the size of the patch/film are controlled according to those of lesions, the lesions which are located in the site difficult to be operated, or the lesions which are too large to be operated can be treated. The radioactive patch/film containing the required amount of radioactivity according to the diseases can be easily made, since the radioactivity of patch/film is in proportion to the size and the amount of the contained particles.

Since the irradiation of the normal site can be prevented by irradiating only the lesion and by blocking the normal site with aluminum foil where the radioactive patch/film are attached, there is no side effect such as damage to bone or cartilage, or over-irradiation of other organs caused by the known method.

And the irradiation of the normal site also can be prevented by using the patch/film blocked on one side in case of the treatment of rubbing part of the body, such as armpit, groin and the like.

In addition, the radioactive patch/film of this invention can be applied in several patterns, wherein the patch is used in monolayer and multilayer and the film is used in spread pattern or tube pattern.

In the following, the process to prepare the radioactive patch/film of this invention will be described.

This invention relates to the radioactive patch/film which contains radioactive materials in a form of tape.

General patches need to satisfy the requirements described as below. To administer medicine into the body through skin, the absorption ratio of effective component is very important since skin prevents foreign substances from absorption, and hence absorption-enhancer should be mixed with adhesive agent. In addition, the effective substance should not react with adhesive agent, absorption-enhancer and the like.

Therefore it is important to choose the adhesive agent, absorption-enhancer and the like satisfying the above requirements according to the effective substance to be administered.

Since the layer of adhesive agent is contact directly with skin for some time, the metabolism of skin is liable to be inhibited, which can provoke side effects such as red spot, dropsy and the like. Hence it is desirable that adhesive agent material does not remain in the lesion site after removal of the tape.

However, in case of the radioactive patch/film, first, absorption enhancer or the like is not necessary since the purpose of the patch/film is only irradiation, not administration of pharmaceutical agents, secondly, the selection of adhesive agent is not limited and most of adhesive agent can be used in this invention, since it is used for irradiation itself by radioisotope and chemical reaction has no effect on irradiation dose. In addition the radioactive material used in this invention does not contaminate environment since radionuclide on the patch/film has short half-life.

The adhesive agents which can be used in this invention are urethane series, acryl series, chloroprene series, polyvinylalcohol (PVA) series, polyvinylchloride (PVC) series, nylon series and the like. Besides, all the adhesive agent to be used for preparing patches can be used in the present invention.

The ratio of radionuclide and adhesive agent is 1-30% in a weight ratio preferably. That is, the concentration of radionuclide is controlled in the range of 1-30% preferably.

The radionuclides which used in this invention can be $\alpha$-emitting radionuclide, $\beta$-emitting radionuclide, $\gamma$-emitting radionuclide and the like. All the radionuclide for the radiation therapy can be within the scope of the present invention. The nuclide can be selected according to the purpose of the treatment.

In particular, $\beta$-emitting radionuclide is preferred in the radioactive patch/film for treating dermal diseases, since $\beta$-emitting isotope has an advantage that it damages only the lesion site, not other tissue due to low permeability. The patch/film emitting $\beta$-ray is an excellent therapeutical agent, since the permeability of $\beta$-ray is so low that it does not reach the normal tissue below the cancer tissue.

In the experiment using $^{166}$Ho-radioactive patch/film, the inventors have found that the excessive irradiation dose damaged only 8mm of tissue on the maximum and cartilage or bones are not damaged at all.

But in case of thick skin cancer $\gamma$-emitting radionuclide is preferred. $^{192}$Ir is $\gamma$-emitting radionuclide by which dermal disease can be treated.

In $\beta$-emitting radionuclides there are $^{198}$Au, $^{90}$Y, $^{186}$Re, $^{32}$P, $^{169}$Er, $^{166}$Ho, $^{153}$Sm, $^{165}$Dy and the like. According to the purpose of the treatment a radionuclide can be selected.

$^{198}$Au also emits $\gamma$-ray abundantly and has a half-life of 2.7 days. $^{32}$P and $^{90}$Y emit only $\beta$-ray without emitting $\gamma$-ray and have a relatively long half-life.

Radionuclides of Lanthane series comprising $^{165}$Dy, $^{166}$Ho, $^{153}$Sm and $^{169}$Er emit $\beta$-ray along with low-energy $\gamma$-ray, and have a very short half-life. They have a merit that the stable nuclides of Lanthane series can be easily converted to radionuclides because they can absorb a lot of neutrons when they are irradiated in the nuclear reactor.

The radioactive patch is prepared according to the steps as follows.

Stable nuclides are made into particles, mixed with adhesive agent, coated on the carrier, laminated for sealing to form the patch, and then the patch is irradiated with neutrons in the nuclear reactor to convert the patch into the radioactive patch. Also, particles of stable nuclide are spread on adhesive tape, laminated for sealing to form the patch, and then irradiated the patch with neutrons in the nuclear reactor to convert into the radioactive patch.

Particles of stable nuclide can be prepared by the established method as follows. Preferably its size is 1-10$\mu$m. To prepare particles of stable nuclide, $^{165}$Ho(NO$_3$)$_3$·5H$_2$O or $^{165}$HoCl$_3$ is dissolved in distilled water, added NaBH$_4$ in sodium hydroxide, precipitated and then the precipitate is made to pieces by ultra-sonication. In addition particles of stable nuclide also can be prepared by use of $^{164}$Dy(NO)$_3$ or $^{164}$DyCl$_3$.

The carrier used in this invention can be paper, textile, metal, plastic film or laminate. Besides, all the carrier can be utilized in the preparation of patches.

The radioactive film is prepared according to the steps as follows.

The stable nuclide is mixed with adhesive agent dissolved in solvent, made into the thin membrane, dried, laminated for sealing to form the film and then irradiated the film with neutrons in the nuclear reactor to convert into the radioactive film. Any stable nuclide compound which is soluble can be used for preparing the radioactive film. Especially $^{164}$Dy(NO$_3$)$_3$ or $^{165}$Ho(NO$_3$)$_3$ is preferred.

As the adhesive agent utilized to prepare the radioactive film, there are urethane series, acryl series, chloroprene series, PVA series, PVC series, nylon series and the like. As the adhesive agent used in this invention elastic one is preferred, since it makes exercise possible even after attachment.

The adhesive agent of this invention should be dissolved in solvent to prepare the radioactive film. Here all the solvent which can dissolve the adhesive agent can be used. Especially dimethyl-formamide or tetrahydrofuran is preferred.

As the sealing agent utilized to prepare the radioactive patch/film, there are polyethylene, cellulose ester, polysulphonate and the like, most preferably polyethylene or its derivative.

The quality and the efficacy of the radioactive patch and the radioactive film are same but the processes of preparation are different a little.

That is, the radioactive patch is prepared by the process, wherein particles of stable nuclide is mixed with adhesive agent, coated on the carrier, laminated with polyethylene film for sealing, and then the patch is irradiated with neutrons in the nuclear reactor. The radioactive film is prepared by the process, wherein stable nuclide is mixed with the solution of adhesive agent, dried, laminated with polyethylene film for sealing, and then irradiated with neutrons in the nuclear reactor.

In the present invention, the radioactive patch/film can be prepared by irradiating the stable-nuclide patch with neutrons in the nuclear reactor after preparing the stable-nuclide patch in advance.

Such a post-irradiation method can reduce the irradiation of the workers.

In the treatment of the radioactive patch/film, preferably 5 Gy (5,000 rad) should be irradiated on tumor lesion. Radiation absorption dose is expressed as formula illustrated as below.

$$D = A\Sigma\Phi i(rk\leftarrow rh)\Delta i$$

$$\triangle i = 5{,}76 \times 10^{-4}\ Ni\ Ei.g.Gy/kBq.hr\ (2.13\ NiEi.g.rad/\mu Ci.hr)$$

where

D : total absorbed dose (kg.Gy (g.rad))
A : accumulated activity (Bq ($\mu$Ci))
$\triangle$ : equilibrium absorbed dose constant
Ni : number emitted per disintegration
Ei : the average energy of the emission
$\Phi i(rk\leftarrow rh) = 1$ in $\beta$ particule

To utilize the above formula, absorption dose is determined according to volume and weight of tissue. The weight of irradiated site is calculated as formula as below.

$$V = \pi r^2 h$$

In this formula r is a radius of patch/film.

Brief Description of the Drawings

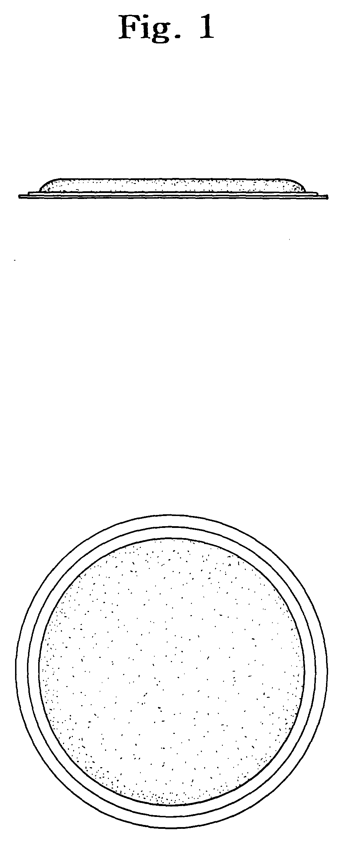

Fig. 1 shows the radioactive patch prepared by the process of this invention.

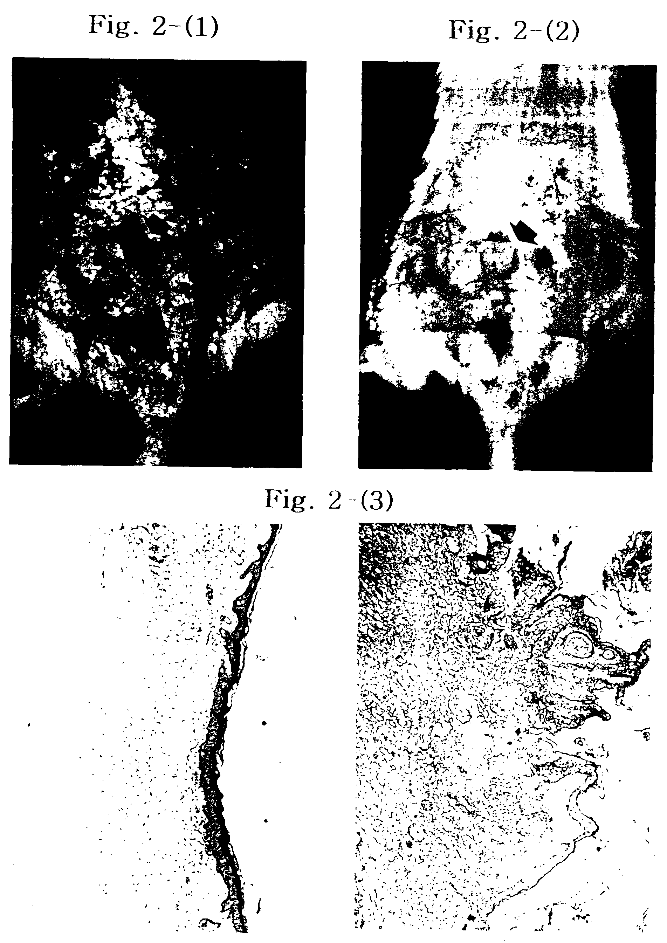

Fig. 2 shows the recovery of tumor by treatment of the radioactive patch of this invention.

1) shows skin cancer in ICR mouse before the treatment.
2) shows $^{166}$Ho-radioactive patch attached on the lesion of skin cancer.
3) shows the recovery of skin cancer after 1 week of the treatment.

The following examples will further illustrate the present invention, which by no means limit the present invention.

**<Example 1> Preparation of $^{166}$Ho-radioactive patch**

1) Preparation of $^{165}$Ho-particle ($^{165}$Ho-macroaggregate, $^{165}$H-MA)

60mg of $^{165}$Ho(No$_3$)$_3$.5H$_2$O or $^{165}$HoCl$_3$ was dissolved in 0.4ml of distilled water, then 200mg of NaBH$_4$ dissolved in 2ml of 2N NaOH was added to produce hydrogen vigorously and to precipitate simultaneously. The precipitate was pulverized by ultra-sonification for 2 minutes to generate 1-5$\mu$m pieces. Then it was centrifuged, washed five times with 5ml of distilled water, also with 5ml of acetone twice and dried in room temperature.

2) Preparation of $^{165}$Ho-patch

$^{165}$Ho particles prepared according to Example 1 (1) was well mixed on a weight basis with 10% adhesive agent (urethane series, acryl series, chloroprene series, polyvinylalcohol series, polyvinylchloride series, nylon series), then the mixture is coated on one side of thin paper, to prepare circular patch of 1cm in diameter. And both side of the patch was laminated with polyethylene film for particles not to leak out of the film.

3) Preparation of $^{166}$Ho-radioactive patch.

$^{165}$Ho-patch prepared according to Example 1 (2) was irradiated as target material in the nuclear reactor to obtain 148-222 MBq (4-6mCi) of the radioactive patch. In the nuclear reactor the neutron flux is $1 \times 10^{13} \sim 1 \times 10^{14}$n/cm$^2$·sec.

### <Example 2> Preparation of $^{165}$Dy-radioactive patch

1) Preparation of $^{164}$Dy-particles

The same method as Example 1 (1) described above was performed using $^{164}$Dy(NO$_3$)$_3$ or $^{164}$DyCl$_3$ compounds.

2) Preparation of $^{164}$Dy-patch

The same method as Example 1 (2) described above was performed using $^{164}$Dy particle prepared according to Example 2 (1) .

3) Preparation of $^{165}$Dy-radioactive patch

The same method as Example 1 (3) was performed using $^{164}$Dy- patch prepared according to Example 2 (2).

### <Example 3> Preparation of $^{90}$Y-radioactive patch

The same method as Example 1 (2), (3) was performed using $^{89}$Y$_2$O$_3$ minute powder.

### <Example 4> Preparation of $^{32}$P-radioactive patch

The same method as Example 1 (2), (3) was performed using red phosphorus minute powder.

### <Example 5> $^{192}$Ir-radioactive patch

The same method as Example 1 was performed using $^{191}$IrCl$_4$ or $^{191}$Ir(IV) compound.

### <Example 6> Preparation of $^{166}$Ho-radioactive film

1) Preparation of $^{165}$Ho-film

Solution in 2ml of dimethyl formamide(DMF) and 10ml of tetrahydrofuran (THF) and 1.2g of polyurethane corresponding to 10%(w/v) of total solution was added and shaken vigorously to dissolve and again 1.2g of $^{165}$Ho (NO$_3$)$_3$ · 5H$_2$O corresponding to 10%(w/v) of total solution was added to dissolve completely. 3ml of solution containing 1.6mg of pure Ho per 1cm$^2$ was spread on 70cm$^2$ flat glass dish which lay on the level. At room temperature the clear solution was left for lhr, dried and then baked in oven at 80°C for 3hrs. After drying $^{165}$Ho-polyurethane membrane was separated from glass dish carefully by needle and pincette and then the circular patch having 1cm in diameter was cut by borer. This patch was laminated on the both side by polyethylene film.

2) Preparation of $^{166}$Ho-radioactive film

$^{165}$Ho-film prepared according to Example 6 (1), which contained 1.2mg of pure Ho having 1cm in diameter, was irradiated as target material in the nuclear reactor to obtain 148-222 MBq (4-6mCi) of the radioactive films respectively. In the nuclear reactor the neutron flux is $1 \times 10^{13} \sim 1 \times 10^{14}$ n/cm$^2$·sec.

### <Example 7> Preparation of $^{165}$Dy-radioactive film

1) Preparation of $^{164}$Dy-film

The same method as Example 6 6 (1) was performed using Dy(NO$_3$)$_3$.

2) Preparation of $^{165}$Dy-radioactive film

The same method as Example 6 6 (2) was performed using $^{164}$Dy-film prepared according to Example 7 (1).

**<Example 8> Stability test of the radioactive patch/film**

Each radioactive patch/film within water of beaker was stirred vigorously by magnetic stirrer and particles or radioactivity leaked out of the patch/film were measured at a time interval by detector. But no particle or no radioactivity was detected in water.

**<Example 9> Efficacy test of the radioactive patch/film**

1) Animal test

Skin cancer was induced to ten ICR mice. 12-o-tetradecanoyl-13-acetate dissolved in 0.1ml of acetone was applied on the skin twice a week and 2'-(4-nitrophenoxy) oxirane dissolved in acetone once a week was applied on the skin for 35 weeks, which induced skin cancer of which the size was about 3-5mm in diameter. Squamous cell carcinomas in 3 mice and keratoacanthomas in 7 mice were developed. The tumor size was 4-8mm in diameter and 3-4mm in thickness.

22,2 MBq (0.6mCi) of $^{166}$Ho-radioactive patch (0.5cm in diameter) was attached to lesion site for 1-2hrs and consequently 70-80 Gy (7,000-8,000 rad) of dose was irradiated. Then the practical radiation absorption dose of tumor was estimated about 45 by measuring TLD. Approximately 3,5 Gy (350 rad) was absorbed by tumor practically. After 1, 4 and 7 weeks, the lesion site of all animals was subjected to pathologic examination. The disappearance of tumor cells, regeneration of epithelial cell and damage of the adjacent tissue by overexposure were examined by H-E staining. After 1 week of exposure, the disappearance of tissue and the protection of normal tissue characterized in β-ray were found remarkably. An acute dermatitis by irradiation was induced, but it was gradually healed with the regeneration of epithelial cell and tumor was identified to disappear completely by pathologic examination. Even by over-exposure, only 8mm of tissue on the maximum was damaged and cartilage and bone below the lesion were never affected.

2) Clinical Test

In two patients with squamous cell carcinoma and basal membrane cancer respectively, $^{166}$Ho-radioactive film containing 333-555MBq (9-15mCi) was attached to the lesion site for 30-45 minutes. Consequently after 1 week of attachment tumor was identified to disappear.

As described above, the radioactive patch/film of this invention is a novel type of radiation pharmaceutical agent. Without expensive equipment, it is utilized conveniently for the radiation therapy. The period of treatment is short, since enough irradiation can be conducted even in one time exposure. And lesions inaccessible by surgical therapy and multiple sites of cancer are also treated by the patch/film of this invention. It is also an outstanding pharmaceutical agent which irradiates only the lesion site without damage of the adjacent normal tissue, since β-ray emitting radionuclides with low penetrative radiation is utilized to irradiate the lesion at the shortest distance.

And the process of preparation can reduce the irradiation of workers, since stable nuclide is converted to radioisotope after completing the preparation of pharmaceutical agent.

The radioactive patch/film is very effective to treat various kinds of cancer. Especially the radioactive patch/film using β-emitting isotope is very effective to treat skin cancer. In the case of thick skin cancer, γ-emitting isotope is also effective.

The radiation dose at one time is different according to disease and its state, but 50-100 Gy (5,000-10,000 rad) is preferable. The radioactive patch/film in which the radiation dose is controlled in the range of 50-100 Gy (5,000-10,000 rad) or 333-555MBq (9-15 mCi), can be attached at the lesion site for about 30 min-3 hrs for the radiation therapy.

**Claims**

**1.** A radioactive patch comprising a carrier, a layer of a mixture of a radionuclide with an adhesive agent, and a laminating layer.

**2.** A radioactive film comprising a layer of a mixture of a radionuclide with an adhesive agent and a laminating layer.

3. The radioactive patch according to Claim 1 or the radioactive film according to Claim 2, characterized in that the radionuclide is a β-rayemitting nuclide or a γ-ray emitting nuclide.

4. The radioactive patch or the radioactive film according to Claim 3, characterized in that the β-ray emitting nuclide is selected from the group comprising $^{153}$Sm, $^{165}$Dy, $^{166}$Ho, $^{32}$P, $^{90}$Y and $^{169}$Er.

5. The radioactive patch or the radioactive film according to Claim 3, characterized in that the γ-ray emitting nuclide is $^{192}$Ir.

6. A process of preparation for the radioactive patch of Claim 1, wherein 1) particles of stable nuclide mixed with adhesive agent are coated on a carrier to prepare a patch, 2) the patch is laminated with a thin membrane for sealing, and 3) the sealed patch is irradiated with neutrons in a nuclear reactor to convert into a radioactive patch emitting radiation.

7. The process of preparation for the radioactive patch according to Claim 6, wherein the particles of stable nuclide are selected from the group comprising $^{165}$Ho compound particle, $^{164}$Dy compound particle, $^{89}$Y$_2$O$_3$ particle, $^{31}$P particle, $^{191}$Ir compound particle.

8. The process of preparation for the radioactive patch according to Claim 6 or 7, characterized in that the size of the particles of stable nuclide is 1-10 μm.

9. A process of preparation for the radioactive film of Claim 2, wherein 1) a solution of stable nuclide mixed with adhesive agent dissolved in solvent is made into a thin membrane and dried to prepare a film, 2) the film is laminated with a thin membrane of laminating material for sealing, 3) then the sealed film is irradiated with neutrons in a nuclear reactor to convert into a radioactive film emitting radiation.

10. The process of preparation for the radioactive film according to Claim 9, wherein the solution of stable nuclide contains $^{165}$Ho(NO$_3$)$_3$ or $^{164}$Dy(NO$_3$)$_3$ dissolved in solvent.

11. The process of preparation for the radioactive patch according to Claim 6 or the radioactive film according to Claim 9, characterized in that the lamination membrane is polyethylene.

12. The radioactive patch of Claim 1 or the radioactive film of Claim 2, for use as a pharmaceutical agent for cancer therapy.

13. The radioactive patch of Claim 1 or the radioactive film of Claim 2, for use as a pharmaceutical agent for the treatment of dermal diseases.

14. The radioactive patch of Claim 1 or the radioactive film of Claim 2, for use as a pharmaceutical agent for the treatment of skin cancer, water-eczema and keloid.


**Patentansprüche**

1. Radioaktives Pflaster enthaltend einen Träger, eine Schicht mit einem Gemisch eines Radionuklids mit einem Haftmittel, und eine Laminierungsschicht.

2. Radioaktiver Film, enthaltend eine Schicht eines Gemisches aus einem Radionuklid mit einem Haftmittel und eine Laminierungsschicht.

3. Radioaktives Pflaster nach Anspruch 1 oder radioaktiver Film nach Anspruch 2, dadurch gekennzeichnet, daß das Radionuklid ein β-Strahlung emittierendes Nuklid oder ein γ-Strahlung emittierendes Nuklid ist.

4. Radioaktives Pflaster oder radioaktiver Film nach Anspruch 3, dadurch gekennzeichnet, daß das β-Strahlung emittierende Nuklid aus einer $^{153}$Sm, $^{165}$Dy, $^{166}$Ho, $^{32}$P, $^{90}$Y, und $^{169}$Er umfassenden Gruppe ausgewählt ist.

5. Radioaktives Pflaster oder radioaktiver Film nach Anspruch 3, dadurch gekennzeichnet, daß das γ-Strahlung emittierende Nuklid $^{192}$Ir ist.

**6.** Verfahren zur Herstellung des radioaktiven Pflasters nach Anspruch 1, wobei 1) Partikel eines stabilen Nuklids im Gemisch mit einem Haftmittel zur Herstellung eines Pflasters auf einen Träger aufgetragen werden, 2) das Pflaster zur Versiegelung mit einer dünnen Membran laminiert wird und 3) das versiegelte Pflaster mit Neutronen in einem Nuklearreaktor bestrahlt wird, um es in ein radioaktives Pflaster, welches Strahlung emittiert, zu überführen.

**7.** Verfahren zur Herstellung des radioaktiven Pflasters nach Anspruch 6, wobei die Partikel des stabilen Nuklids aus einer $^{165}$Ho Verbindungspartikel, $^{164}$Dy Verbindungspartikel, $^{89}$Y$_2$O$_3$ Partikel, $^{31}$P Partikel, $^{191}$Ir Verbindungspartikel umfassenden Gruppe ausgewählt sind.

**8.** Verfahren zur Herstellung des radioaktiven Pflasters nach Anspruch 6 oder 7, dadurch gekennzeichnet, daß die Partikelgröße des stabilen Nuklids 1-10 μm beträgt.

**9.** Verfahren zur Herstellung des radioaktivem Films nach Anspruch 2, wobei 1) eine Lösung eines stabilen Nuklids, vermischt mit einem Haftmittel, welches in einem Lösungsmittel gelöst ist, zu einer dünnen Membran verarbeitet und zur Filmherstellung getrocknet wird, 2) der Film mit einer dünnen Membran aus Laminierungsmaterial zur Versiegelung laminiert wird, 3) dann der versiegelte Film in einem Nuklearreaktor mit Neutronen bestrahlt wird, um diesen in einen Strahlung emittierenden radioaktiven Film umzuwandeln.

**10.** Verfahren zur Herstellung des radioaktiven Films nach Anspruch 9, worin die Lösung des stabilen Nuklids $^{165}$Ho (NO)$_3$ oder $^{164}$Dy(NO)$_3$ gelöst in einem Lösungsmittel enthält.

**11.** Verfahren zur Herstellung des radioaktiven Pflasters nach Anspruch 6 oder des radioaktiven Films nach Anspruch 9, dadurch gekennzeichnet, daß die Membran zur Laminierung aus Polyethylen besteht.

**12.** Radioaktives Pflaster nach Anspruch 1 oder radioaktiver Film nach Anspruch 2 zur Verwendung als pharmazeutisches Mittel in der Krebstherapie.

**13.** Radioaktives Pflaster nach Anspruch 1 oder radioaktiver Film nach Anspruch 2 zur Verwendung als pharmazeutisches Mittel für die Behandlung von Hauterkrankungen.

**14.** Radioaktives Pflaster nach Anspruch 1 oder radioaktiver Film nach Anspruch 2 für die Verwendung als pharmazeutisches Mittel zur Behandlung von Hautkrebs, Wasserekzem und Narbengeschwulsten.

**Revendications**

**1.** Timbre radioactif comprenant un support, une couche d'un mélange d'un radionucléide avec un agent adhésif et une couche de stratification.

**2.** Film radioactif comprenant une couche d'un mélange d'un radionucléide avec un agent adhésif et une couche de stratification.

**3.** Timbre radioactif selon la revendication 1 ou film radioactif selon la revendication 2, caractérisés en ce que le radionucléide est un nucléide émettant des rayons β ou un nucléide émettant des rayons γ.

**4.** Timbre radioactif ou film radioactif selon la revendication 3, caractérisé en ce que le nucléide émettant des rayons β est choisi dans le groupe constitué par $^{153}$Sm, $^{165}$Dy, $^{166}$Ho, $^{32}$P et $^{169}$Er.

**5.** Timbre radioactif ou film radioactif selon la revendication 3, caractérisé en ce que le nucléide émettant des rayons γ est $^{192}$Ir.

**6.** Procédé de préparation du timbre radioactif selon la revendication 1, dans lequel 1) on dépose une couche de particules d'un nucléide stable mélangé avec un agent adhésif sur un support pour préparer un timbre, 2) on soumet le timbre à une stratification avec une membrane mince pour le scellage, et 3) on irradie le timbre scellé avec des neutrons dans un réacteur nucléaire pour le transformer en un timbre radioactif émettant un rayonnement.

**7.** Procédé de préparation du timbre radioactif selon la revendication 6, dans lequel les particules de nucléide stable sont choisies dans le groupe constitué par des particules d'un composé de $^{165}$Ho, des particules d'un composé

de $^{164}$Dy, des particules de $^{89}$Y$_2$O$_3$, des particules de $^{31}$P et des particules d'un composé de $^{191}$Ir.

8. Procédé de préparation du timbre radioactif selon la revendication 6 ou 7, caractérisé en ce que la taille des particules du nucléide stable est de 1 à 10 μm.

9. Procédé de préparation du film radioactif selon la revendication 2, dans lequel 1) on transforme une solution d'un nucléide stable mélangé avec un agent adhésif dans un solvant en une membrane mince que l'on sèche pour préparer un film, 2) on soumet le film à une stratification avec une membrane mince d'un matériau de stratification pour le scellage, 3) on irradie ensuite le film scellé avec des neutrons dans un réacteur nucléaire pour le transformer en un film radioactif émettant un rayonnement.

10. Procédé de préparation du film radioactif selon la revendication 9, dans lequel la solution de nucléide stable contient du $^{165}$Ho(NO$_3$)$_3$ ou du $^{164}$Dy(NO$_3$)$_3$ dissous dans un solvant.

11. Procédé de préparation du timbre radioactif selon la revendication 6 ou du film radioactif selon la revendication 9, caractérisé en ce que la membrane de stratification est constituée de polyéthylène.

12. Timbre radioactif selon la revendication 1 ou film radioactif selon la revendication 2, à utiliser comme agent pharmaceutique pour la thérapie du cancer.

13. Timbre radioactif selon la revendication 1 ou film radioactif selon la revendication 2, à utiliser comme agent pharmaceutique pour le traitement de maladies de la peau.

14. Timbre radioactif selon la revendication 1 ou film radioactif selon la revendication 2, à utiliser comme agent pharmaceutique pour le traitement du cancer de la peau, de l'eczéma suintant et de chéloïdes.

# Fig. 1

Fig. 2-(1)

Fig. 2-(2)

Fig. 2-(3)